(19) **Europäisches Patentamt**
**European Patent Office**
**Office européen des brevets**

(11) **EP 3 778 013 A1**

(12) **EUROPEAN PATENT APPLICATION**
published in accordance with Art. 153(4) EPC

(43) Date of publication:
**17.02.2021 Bulletin 2021/07**

(21) Application number: **19765961.8**

(22) Date of filing: **11.03.2019**

(51) Int Cl.:
**B01J 23/887** (2006.01)  **C07C 253/26** (2006.01)
**C07C 255/08** (2006.01)  **C07B 61/00** (2006.01)

(86) International application number:
**PCT/JP2019/009654**

(87) International publication number:
**WO 2019/198401 (17.10.2019 Gazette 2019/42)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **13.04.2018  JP 2018077658**

(71) Applicant: **ASAHI KASEI KABUSHIKI KAISHA
Tokyo 100-0006 (JP)**

(72) Inventors:
• **TOMODA, Atsushi**
**Tokyo**
**1000006 (JP)**
• **FUKUZAWA, Akiyoshi**
**Tokyo**
**1000006 (JP)**

(74) Representative: **dompatent von Kreisler Selting
Werner -
Partnerschaft von Patent- und Rechtsanwälten
mbB
Deichmannhaus am Dom
Bahnhofsvorplatz 1
50667 Köln (DE)**

(54) **CATALYST, METHOD FOR PRODUCING CATALYST, AND METHOD FOR PRODUCING ACRYLONITRILE**

(57)    The present invention provides a catalyst comprising molybdenum, bismuth, and iron, wherein a reduction rate is in a range of 0.20 to 5.00%.

EP 3 778 013 A1

**Description**

Technical Field

The present invention relates to a catalyst, a method for producing the catalyst, and a method for producing acrylonitrile.

Background Art

As a method for producing acrylonitrile, a method in which propylene is subjected to ammoxidation is known. Through this ammoxidation, acrylonitrile and hydrogen cyanide can be obtained at the same time.

As an ammoxidation catalyst, an oxide catalyst comprising molybdenum, bismuth, and iron, or an oxide catalyst comprising antimony and iron are utilized and various improvements have been made to catalysts having these basic compositions, for improving the efficiency of ammoxidation reaction.

For example, it is considered that a fluidized bed ammoxidation reaction catalyst represented by the following formula (1) disclosed in Patent Literature 1 can produce acrylonitrile in a high yield and stably for a long period without using an excess amount of ammonia in ammoxidation of propylene.

$$Mo_{12}Bi_aFe_bNi_cCO_dCe_eCr_fX_gO_h/(SiO_2)_A... \qquad (1)$$

wherein Mo represents molybdenum; Bi represents bismuth; Fe represents iron; Ni represents nickel; Co represents cobalt; Ce represents cerium; Cr represents chrome; X represents at least one element selected from the group consisting of potassium, rubidium, and cesium; $SiO_2$ represents silica; a, b, c, d, e, f, g, and h each represents an atomic ratio of each element and satisfy $0.1 \leq a \leq 1$, $1 \leq b < 3$, $1 \leq c \leq 6.5$, $1 \leq d \leq 6.5$, $0.2 \leq e \leq 1.2$, $f \leq 0.05$, and $0.05 \leq g \leq 1$, respectively; h represents an atomic ratio of oxygen atoms which satisfy atomic valence of each constituent element except for silica; A represents a content of silica in the complex (% by mass) and satisfies $35 \leq A \leq 48$; and values of $\alpha$, $\beta$, and $\gamma$ obtained from atomic ratios of each element by using the following expression (2), (3), and (4) satisfy $0.03 \leq \alpha \leq 0.08$, $0.2 \leq \beta \leq 0.4$, and $0.5 \leq \gamma \leq 2$.

$$\alpha = 1.5a/(1.5(b+f)+c+d)....(2)$$

$$\beta = 1.5(b+f)/(c+d).........(3)$$

$$\gamma = d/c...................(4)$$

Citation List

Patent Literature

[Patent Literature 1] WO2017-130906

Summary of Invention

Technical Problem

In the ammoxidation of propylene, the productivity of not only acrylonitrile but also hydrogen cyanide is required to be increased and improving the yield of hydrogen cyanide while maintaining a high yield of acrylonitrile is desired.

In addition, in the production of acrylonitrile and hydrogen cyanide, performing continuous reaction for a long time may cause lowering of the performance of the catalyst. Consequently, the catalyst may additionally be added to a reaction system without stopping the reaction, in order to allow continuous production of acrylonitrile and hydrogen cyanide. Thus, in order to maintain the productivity of acrylonitrile and hydrogen cyanide upon addition of the catalyst to the reaction system, a catalyst having a performance as an ammoxidation catalyst and a high reaction activity of propylene (hereinafter, also referred to as the catalyst activity) is required.

The present invention has been completed in consideration of the problems, and an object of the present invention is to provide a catalyst that can improve the yield of hydrogen cyanide while maintaining the high yield of acrylonitrile in the ammoxidation of propylene and has a high reaction activity of propylene.

Solution to Problem

The present inventors have conducted intensive studies to solve the above problems and found that a catalyst comprising a certain metallic species and having a reduction rate in a certain range can improve the yield of hydrogen cyanide while maintaining a high yield of acrylonitrile that is a product of the ammoxidation of propylene, and has a high catalyst activity, thereby completed the present invention.

That is, the present invention is as follows.

[1] A catalyst comprising molybdenum, bismuth, and iron, wherein
a reduction rate is in a range of 0.20 to 5.00%.
[2] The catalyst according to [1], wherein the reduction rate is in a range of 0.70 to 4.30%.
[3] The catalyst according to [1] or [2], wherein the catalyst comprises a complex metal oxide having a composition represented by the following formula (1):

$$MO_{12}Bi_aFe_bX_cY_dZ_eO_f \qquad (1)$$

wherein, X represents at least one element selected from the group consisting of nickel, cobalt, magnesium, calcium, zinc, strontium, barium, and tungsten;
Y represents at least one element selected from the group consisting of cerium, chromium, lanthanum, neodymium, yttrium, praseodymium, samarium, aluminum, boron, gallium, and indium;
Z represents at least one element selected from the group consisting of sodium, potassium, rubidium, and cesium;
a, b, c, d, and e satisfy $0.1 \leq a \leq 2.0$, $0.1 \leq b \leq 2.8$, $0.1 \leq c \leq 0.0$, $0.1 \leq d \leq 3.0$, and $0.01 \leq e \leq 2.0$, respectively; and
f represents a number of oxygen atom needed to satisfy an atomic valence requirement of element existing other than oxygen.

[4] A method for producing the catalyst according to any one of [1] to [3] comprising:

a contacting step of bringing a calcined product comprising molybdenum, bismuth, and iron into contact with propylene, air, and ammonia, wherein
the contacting step comprises:

a step of setting a molar ratio of ammonia/propylene to a condition of greater than 2.50 (condition 1); and
a step of setting a molar ratio of ammonia/propylene to a condition of 2.50 or less (condition 2).

[5] The method for producing the catalyst according to [4], wherein
the molar ratio of ammonia/air in the condition 1 is greater than 0.12, and
the molar ratio of ammonia/air in the condition 2 is 0.12 or less.
[6] A method for producing acrylonitrile comprising a step of reacting propylene, molecular oxygen, and ammonia with each other in the presence of the catalyst according to any one of [1] to [3].
[7] The method for producing acrylonitrile according to [6], wherein the method is carried out by a fluidized bed reactor.
[8] The method for producing acrylonitrile according to [6] or [7], wherein a molar ratio of ammonia and air to propylene (propylene/ammonia/air) is in a range of 1.0/(0.8 to 2.5)/(7.0 to 12.0).
[9] The method for producing acrylonitrile according to any one of [6] to [8], wherein a reaction is carried out in a temperature range of 300 to 500°C.

The catalyst of the present invention can improve the yield of hydrogen cyanide while maintaining a high yield of acrylonitrile that is a product of the ammoxidation of propylene and increase the reaction activity of propylene in the reaction system. Therefore, a production method comprising a step of subjecting propylene to ammoxidation in the presence of the catalyst of the present invention can increase the productivity of acrylonitrile and hydrogen cyanide and efficiently supply acrylonitrile and hydrogen cyanide.

Description of Embodiments

Hereinafter, an embodiment of the present invention (hereinafter, referred to as the "present embodiment") will be described in detail. The present invention is not limited to the following embodiment and can be variously modified within the scope thereof. In the present description, when the numerical value or physical property value is represented by using "to" by describing before and after the "to", values described before and after the "to" are included. For example, the description of a numerical range of "1 to 100" encompasses both its upper limit value "100" and its lower limit value "1". The same applies to the descriptions of other numerical ranges.

A catalyst of the present embodiment comprises molybdenum, bismuth, and iron. In addition, a reduction rate of the catalyst of the present embodiment is in a range of 0.20 to 5.00%.

By using the catalyst of the present embodiment in ammoxidation of propylene, the yield of hydrogen cyanide can be improved while maintaining the high yield of acrylonitrile that is a product of the ammoxidation of propylene. In addition, the catalyst of the present embodiment has a high catalyst activity and when being used as the catalyst to be added to the ammoxidation reaction system of propylene, propylene reaction activity of the catalyst in a reactor which is lowered by long-term operation can be increased.

The reduction rate of the catalyst of the present embodiment is 0.20 to 5.00%, preferably 0.70 to 4.30%, and more preferably 1.00 to 3.70%.

By setting the reduction rate to 0.20% or more and 5.00% or less, the yield of hydrogen cyanide is improved while maintaining the high yield of acrylonitrile and the catalyst activity is increased.

Examples of a method for setting the reduction rate to 0.20 to 5.00% include a method for controlling a molar ratio of ammonia/propylene in a step of preparing the catalyst, as described in Examples described later.

Specifically, the reduction rate can be measured by the method described in Examples.

The catalyst of the present embodiment is not particularly limited, as long as it comprises at least molybdenum (Mo), bismuth (Bi), and iron (Fe), and the catalyst of the present embodiment may comprise other elements.

Examples of the other elements include magnesium and alkali metals.

For example, by comprising magnesium, the crystal phase can be stabilized and there is a tendency that $\alpha$-transformation of crystal phase, which may lead to a decrease in performance when the catalyst is subjected to fluidized bed reaction, is suppressed. By comprising alkali metal, there is a tendency that production of a by-product is suppressed, and a calcination temperature of the catalyst is held in a preferable range.

The catalyst of the present embodiment preferably comprises a complex metal oxide having a composition represented by formula (1).

$$Mo_{12}Bi_aFe_bX_cY_dZ_eO_f \qquad (1)$$

(wherein, X represents at least one element selected from the group consisting of nickel, cobalt, magnesium, calcium, zinc, strontium, barium, and tungsten;

Y represents at least one element selected from the group consisting of cerium, chromium, lanthanum, neodymium, yttrium, praseodymium, samarium, aluminum, boron, gallium, and indium;

Z represents at least one element selected from the group consisting of sodium, potassium, rubidium, and cesium;

a, b, c, d, and e satisfy $0.1{\leq}a{\leq}2.0$, $0.1{\leq}b{\leq}2.8$, $0.1{\leq}c{\leq}0.0$, $0.1{\leq}d{\leq}3.0$, and $0.01{\leq}e{\leq}2.0$, respectively; and

f represents a number of oxygen atom needed to satisfy an atomic valence requirement of element existing other than oxygen.)

The atomic ratio a of bismuth to 12 atoms of molybdenum is $0.1{\leq}a{\leq}2.0$, and preferably $0.2{\leq}a{\leq}1.8$.

By setting a to 0.1 or more and 2.0 or less, there is a tendency that yields of producing acrylonitrile and hydrogen cyanide in the early stage of the reaction increase and the stability of reaction becomes excellent.

The atomic ratio b of iron to 12 atoms of molybdenum is $0.1{\leq}b{\leq}2.8$, and preferably $0.2{\leq}b{\leq}2.6$.

The atomic ratio c of element X to 12 atoms of molybdenum is $0.1{\leq}c{\leq}0.0$, and preferably $0.2{\leq}c{\leq}9.6$. Element X is at least one selected from the group consisting of nickel, cobalt, magnesium, calcium, zinc, strontium, barium, and tungsten.

The atomic ratio d of element Y to 12 atoms of molybdenum is $0.1{\leq}d{\leq}3.0$, and preferably $0.2{\leq}d{\leq}2.8$. Element Y is at least one selected from the group consisting of cerium, chromium, lanthanum, neodymium, yttrium, praseodymium, samarium, aluminum, boron, gallium, and indium. Element Y preferably comprises at least cerium and may further comprise one or more elements selected from the group consisting of chromium, lanthanum, neodymium, yttrium, praseodymium, samarium, aluminum, gallium, and indium.

The atomic ratio e of element Z to 12 atoms of molybdenum is $0.01{\leq}e{\leq}2.0$, and preferably $0.03{\leq}e{\leq}1.8$. Element Z is at least one selected from the group consisting of sodium, potassium, rubidium, and cesium.

In addition, the atomic ratio f of oxygen to 12 atoms of molybdenum may be a number of oxygen atom needed to satisfy an atomic valence requirement of element existing other than oxygen.

The catalyst of the present embodiment may be made by carrying on a carrier. As the carrier, an oxide such as silica, alumina, titania, or zirconia is used. Silica is preferable from the viewpoint that lowering of the selectivity of the object is small, and the wear resistance and particle strength of the formed particles of the catalyst are good. That is, one of the preferred aspects of the catalyst of the present embodiment is the catalyst further comprising silica.

The amount of a silica carrier used is in a range of 20% by mass to 80% by mass, preferably 30% by mass to 70% by mass, and further preferably 40% by mass to 60% by mass based on the total mass of the silica carrier and the complex metal oxide.

The shape and particle size of the catalyst of the present embodiment are not particularly limited and when used as a fluidized bed catalyst, the catalyst has preferably a spherical shape and preferably has a particle diameter of 10 to 180 $\mu$m, from the viewpoint of fluidity.

[Method for producing Catalyst]

The catalyst of the present embodiment is produced by the production method comprising a step of bringing a calcined product comprising molybdenum, bismuth, and iron into contact with propylene, air, and ammonia, and the step comprises steps of setting to the following condition 1 and condition 2.

The condition 1 is a step of setting the molar ratio of ammonia/propylene to greater than 2.50. The condition 2 is a step of setting the molar ratio of ammonia/propylene to 2.50 or less.

The molar ratio of ammonia/propylene in the condition 1 is a value greater than 2.50, and preferably a value greater than 5.00. By setting the molar ratio of ammonia/propylene in the condition 1 to a value greater than 2.50, there is a tendency that the yield of hydrogen cyanide can be improved while maintaining the high yield of acrylonitrile that is a product of the ammoxidation of propylene and the catalyst having a high catalyst activity can be obtained. The upper limit value of the molar ratio of ammonia/propylene in the condition 1 is not particularly limited and may be typically a value of 30.00 or less, a value of 25.00 or less, and a value of 20.00 or less.

In addition, the molar ratio of ammonia/air in the condition 1 is preferably a value greater than 0.12, and more preferably a value greater than 0.21. By setting the molar ratio of ammonia/air in the condition 1 to a value greater than 0.12, there is a tendency that the yield of hydrogen cyanide can be improved while maintaining the high yield of acrylonitrile that is a product of the ammoxidation of propylene and the catalyst having a high catalyst activity can be obtained. The upper limit value of the molar ratio of ammonia/air in the condition 1 is not particularly limited and may be typically a value of 1.00 or less, a value of 0.70 or less, and a value of 0.50 or less.

The molar ratio of ammonia/propylene in the condition 2 is a value of 2.50 or less, and preferably a value of 2.00 or less. By setting the molar ratio of ammonia/propylene in the condition 2 to a value of 2.50 or less, there is a tendency that the yield of hydrogen cyanide can be improved while maintaining the high yield of acrylonitrile that is a product of the ammoxidation of propylene and the catalyst having a high catalyst activity can be obtained. The lower limit value of the molar ratio of ammonia/propylene in the condition 2 is not particularly limited and may be typically a value greater than 0, a value greater than 0.1, and a value greater than 0.5.

The molar ratio of ammonia/air in the condition 2 is preferably a value of 0.12 or less, and more preferably a value of 0.10 or less. By setting the molar ratio of ammonia/air in the condition 2 to a value of 0.12 or less, there is a tendency that the yield of hydrogen cyanide can be improved while maintaining the high yield of acrylonitrile that is a product of the ammoxidation of propylene and the catalyst having a high catalyst activity can be obtained. The lower limit value of the molar ratio of ammonia/air in the condition 2 is not particularly limited and may be typically a value greater than 0, a value greater than 0.01, and a value greater than 0.05.

The calcined product comprising molybdenum, bismuth, and iron can be produced with reference to a known method, for example, the production method described in WO2018/211858. Specifically, the calcined product comprising molybdenum, bismuth, and iron is not particularly limited and can be produced by a method comprising a step of spray-drying a slurry comprising molybdenum, bismuth, and iron to obtain a dried particle and a step of calcining the dried particle in the air, for example. The calcined product comprising molybdenum, bismuth, and iron may comprise not only molybdenum, bismuth, and iron, but also metals contained in the composition represented by formula (1). The calcined product comprising molybdenum, bismuth, and iron is preferably a complex metal oxide having the composition represented by formula (1).

The slurry comprising molybdenum, bismuth, and iron can be obtained by mixing a starting material of the catalyst and a solvent. The solvent is preferably water, and the slurry is preferably an aqueous slurry. When the catalyst of the present embodiment is carried on silica, a preparation method is preferably used in which an aqueous solution containing molybdenum is mixed with an aqueous solution containing silica and stirred, followed by mixing with a solution containing bismuth and other metals and stirring.

As the starting material of silica, silica sol is preferable. A preferable concentration of silica sol being in a state of a starting material containing no other metal components is 10 to 50% by mass.

The starting materials for each element constituting catalysts, such as molybdenum, bismuth, cerium, iron, nickel, cobalt, magnesium, zinc, potassium, rubidium, and cesium that are used for preparing the slurry may be a salt soluble in water or nitric acid, and examples thereof include ammonium salt, nitrate, hydrochloride, sulfate, organic acid salt of each metal.

The ammonium salt is preferably used as the starting material comprising molybdenum, and the nitrate is preferably used as the starting material comprising bismuth, cerium, iron, nickel, magnesium, zinc, potassium, rubidium, and cesium.

The slurry comprising molybdenum, bismuth, and iron is spray-dried to prepare the dried particle.

In spray-drying, the slurry is spray-dried to obtain the spherical particle. The spraying of the aqueous slurry is performed by industrially and typically used methods such as a centrifugal method, a two-fluid nozzle method, and a high-pressure nozzle method, and the spraying is preferably performed by the centrifugal method. Heated air is preferably used for drying and examples of a heat source for drying include steam and electric heater. The inlet temperature of the drier is preferably 100°C to 400°C, and more preferably 150°C to 300°C. The outlet temperature of the drier is preferably 100°C to 180°C, and more preferably 120°C to 170°C.

The dried particle obtained as described above is calcined in the air to obtain the calcined product.

The calcination is performed by using a typical tunnel-type or rotary-type kiln. The calcination temperature is preferably in a range of 500 to 750°C, and more preferably 500 to 680°C. The calcination time may be appropriately adjusted according to the calcination temperature and is preferably in a range of 1 to 20 hours.

The size of the calcined product of the present embodiment is not particularly limited and the calcined product is preferably spherical and preferably has a particle diameter of 10 to 180 $\mu$m.

The calcined product obtained as described above is subjected to bringing into contact with propylene, ammonia, and air, and a fluidized bed reactor can be suitably used for the contact.

The fluidized bed reactor is not particularly limited, but a reactor that is preferably a vertical cylindrical type reactor comprising an air dispersing plate, a starting material gas dispersion pipe for supplying propylene and ammonia provided on the air dispersing plate, a reactor outlet and the like can be suitably used.

Specifically, the contact with propylene, ammonia, and air by using the fluidized bed reactor can be performed as follows.

The following steps A and B are treatment steps performed before conditions 1 and 2 described below and performed as necessary.

Step A: first, a calcined product comprising molybdenum, bismuth, and iron are packed into the fluidized bed reactor, air is supplied from the air dispersing plate at a flow rate of 500 to 100000 $Nm^3$/hr, nitrogen is supplied from the starting material gas dispersion pipe at a flow rate of 500 to 10000 $Nm^3$/hr, and the temperature in the reactor is set to 350 to 550°C.

Step B: next, the flow rate of the air is lowered to 30 to 70% of 500 to 100000 $Nm^3$/hr and ammonia is supplied from the starting material gas dispersion pipe to make the flow rate 500 to 10000 $Nm^3$/hr.

Next, as the condition 1, propylene is supplied from the starting material gas dispersion pipe. On that occasion, the flow rate is set so that the molar ratio of ammonia/propylene can be greater than 2.5 and the molar ratio of ammonia/air can be greater than 0.12.

Thereafter, as the condition 2, the flow rate is set so that the molar ratio of ammonia/propylene can be 2.5 or less and the molar ratio of ammonia/air can be 0.12 or less.

Then, the supply of nitrogen is stopped.

By undergoing these conditions 1 and 2, the catalyst of the present embodiment can be obtained.

The duration of keeping the condition 1 is typically 0.1 to 5.0 hours, and preferably 0.5 to 3.0 hours.

The duration of keeping the condition 2 is not particularly limited, may be appropriately adjusted, and is typically 0.1 hours or more, and preferably 0.5 hours or more. The duration of keeping the condition 2 is not particularly limited and may be appropriately adjusted. In addition, after undergoing the condition 2, acrylonitrile and hydrogen cyanide may be continuously produced and on that occasion, the flow rate may be set so that the molar ratio of ammonia/propylene can be 2.5 or less and the molar ratio of ammonia/air can be 0.12 or less, that is, the condition 2 may be continued. The condition 2 can be continued along with the production of acrylonitrile and hydrogen cyanide, as described above, and the duration of condition 2 may be typically 20 hours or less and 10 hours or less.

The temperature in the reactor in the condition 1 is typically 350 to 550°C and the temperature in the reactor in the condition 2 is typically 300 to 500°C. The pressure in the reactor under conditions 1 and 2 is typically 0.01 to 1.00 MPa at the top of the reactor.

[Method for Producing Acrylonitrile and Hydrogen cyanide]

The method for producing acrylonitrile according to the present embodiment uses the catalyst of the present embodiment. That is, the method for producing acrylonitrile according to the present embodiment comprises a step of reacting propylene, molecular oxygen, and ammonia with each other in the presence of the catalyst of the present embodiment. The production method of the present embodiment is preferably performed by fluidized bed ammoxidation reaction. In addition, the production of acrylonitrile of the present embodiment can be performed in the same reactor as the fluidized bed reactor used for producing the catalyst as described above.

By the production method according to the present embodiment, acrylonitrile and hydrogen cyanide can be produced.

The method for producing acrylonitrile according to the present embodiment may be performed, for example, in the fluidized bed reactor typically used. Propylene and ammonia each being a starting material are not necessarily of high purity and propylene and ammonia of industrial grade can be used. As the molecular oxygen source, air is typically preferably used and a gas whose oxygen concentration is increased by mixing oxygen with air can be used.

When the molecular oxygen source in the method for producing acrylonitrile according to the present embodiment is air, a composition of a starting material gas (a molar ratio of ammonia and air to propylene; propylene/ammonia/air) is preferably in the range of 1/(0.8 to 2.5)/(7.0 to 12.0), and more preferably in the range of 1/(0.9 to 1.3)/(8.0 to 11.0).

The reaction temperature in the method for producing acrylonitrile according to the present embodiment is preferably in the range of 300 to 500°C, and more preferably in the range of 400 to 480°C. The reaction pressure is preferably in the range of normal pressure to 0.3 MPa. The contact time of the starting material gas with the catalyst is preferably 0.5 to 20 (sec-g/cc), and more preferably 1 to 10 (sec-g/cc).

Examples

Hereinafter, the present embodiment will be described in more detail giving Examples, but the present embodiment is in no way limited to Examples described below. In addition, evaluation methods of each physical property are as described below.

[Reduction Rate Measurement]

The measurement of the reduction rate was performed by the following procedure.

First, into a 300 mL beaker, 5 mL of purified water and 1.4 g of the catalyst produced in each of Examples and Comparative Examples were added and 5 mL of sulfuric acid aqueous solution (water volume:sulfuric acid volume = 1:1) was added.

Next, 24 mL of 0.005 mol/L potassium permanganate aqueous solution was added and 10 mL of sulfuric acid aqueous solution (water volume:sulfuric acid volume = 1:1) was further added thereto. Further, the purified water was added until the total liquid volume became 150 mL and heated in a water bath at 73°C for 1 hour, thereafter, the mixture was filtered with a filter paper to collect the filtrate.

After adding 15 mL of 0.0125 mol/L sodium oxalate solution to the filtrate, the mixture was heated in the water bath at 73°C for 10 minutes and 2 mL of sulfuric acid aqueous solution (water volume:sulfuric acid volume = 1:1) was added.

Thereafter, the filtrate was titrated with 0.005 mol/L of potassium permanganate aqueous solution and the amount of potassium permanganate titrated (mL) was recorded by setting the condition when the filtrate becomes brown as the end point.

The reduction rate was calculated by the following expression based on the amount of addition of potassium permanganate (24 mL), the amount of addition of sodium oxalate (15 mL), the amount of substance of oxygen per 1 electron (8), and the numerical value for matching the unit (40).

$$\text{Reduction rate (\%)} = \frac{((24 + \text{Titrated amount of potassium permanganate}) - 15) \times 8 \times 100}{40 \times 1000 \times (1.4 \times A \times (1 - (\text{Silica sol concentration}/100)))}$$

A in the expression for calculating the reduction rate is a proportion of the amount of substance of oxygen in a metal oxide molecule. In addition, $M_{metalOf}$ in the following expression is the amount of substance of the metal oxide according to the atomic ratio of each metal and $M_{metal}$ is the amount of substance of each metal.

$$A = \frac{((M_{Mo12Of} + M_{BiaOf} + M_{FebOf} + M_{XcOf} + M_{YdOf} + M_{ZeOf}) - (M_{Mo12} + M_{Bia} + M_{Feb} + M_{Xc} + M_{Ydf} + M_{ze}))}{(M_{Mo12Of} + M_{BiaOf} + M_{FebOf} + M_{XcOf} + M_{YdOf} + M_{ZeOf})}$$

[Propylene Conversion Rate, Acrylonitrile Yield, Hydrogen Cyanide Yield]

By using the catalyst obtained in Examples and Comparative Examples, acrylonitrile and hydrogen cyanide were produced by ammoxidation reaction of propylene. A Pyrex (R) glass pipe having an inner diameter of 25 mm and having 16 of 10-mesh wire nets built-in at an interval of 1 cm was used as a reaction pipe to be used in the ammoxidation reaction.

The reaction was carried out by setting the amount of the catalyst to 50 cc, the reaction temperature to 430°C and the reaction pressure to 0.17 MPa, and supplying a mixed gas of propylene/ammonia/air at 250 to 450 cc/sec (in terms of NTP) as the total gas flow rate. On that occasion, a propylene content in the mixed gas was set to 9% by volume and a molar ratio of propylene/ammonia/air was set to 1/(0.7 to 2.5)/(8.0 to 13.5). Within this range, an ammonia flow rate was appropriately changed such that the sulfuric acid unit requirement defined by the following expression was 20±2 kg/T-AN, and an air flow rate was appropriately changed such that an oxygen concentration of a gas at an outlet of a reactor was 0.2±0.02% by volume. In addition, the contact time defined by the following expression was changed by changing the flow rate of the total mixed gas and set such that the conversion rate of propylene defined by the following expression was 99.3±0.2%.

The acrylonitrile yield and the hydrogen cyanide yield produced through the reaction were determined as a value defined by the following expression.

$$\text{Sulfuric acid unit requirement (kg/T-AN)} = \frac{\text{Weight for sulfuric acid needed to neutralize unreacted ammonia (kg)}}{\text{Weight of acrylonitrile produced (T)}}$$

$$\text{Contact time (sec.)} = \frac{\text{Amount of catalyst (cc)}}{\text{Flow rate of mixed gas (cc} - \text{NTP/sec)}} \times \frac{273}{273 + \text{reaction temperature (}^{\circ}\text{C)}} \times \frac{\text{Reaction pressure (MPa)}}{0.10}$$

$$\text{Conversion rate of propylene (\%)} = \frac{\text{Propylene consumed (mol)}}{\text{Propylene supplied (mol)}} \times 100$$

$$\text{Acrylonitrile yield (\%)} = \frac{\text{Acrylonitrile produced (mol)}}{\text{Propylene supplied (mol)}} \times 100$$

$$\text{Hydrogen cyanide yield (\%)} = \frac{\text{Hydrogen cyanide produced (mol)}}{\text{Propylene supplied (mol)}} \times 100$$

[Catalyst Activity]

The catalyst activity represents the level of the activity of the catalyst and represented by a reaction rate calculated from the conversion rate of propylene determined by the method described above.

By using each catalyst obtained in Examples and Comparative Examples, acrylonitrile and hydrogen cyanide were produced by ammoxidation reaction of propylene. As a reaction pipe for use in the above ammoxidation reaction, a reaction pipe having an inner diameter of 10 mm manufactured by SUS316 was used.

The reaction was carried out by setting the amount of the catalyst to 1 cc, the reaction temperature to 440°C, and the reaction pressure to normal pressure, and supplying 40 cc/sec (in terms of NTP) of a mixed gas of propylene/ammonia/oxygen/helium as the total gas flow rate. On that occasion, the propylene content in the mixed gas was set to 5.4% by volume and a molar ratio of propylene/ammonia/oxygen was set to 1/1.2/1.89 and helium was set to the flow rate such that the total gas flow rate becomes 40 cc/sec (in terms of NTP). The contact time defined by the expression described above was calculated from the flow rate of the mixed gas and the propylene conversion rate defined by the expression described above was calculated from the value of propylene supplied and consumed.

From these contact time and propylene conversion rate, the catalyst activity can be determined by the following expression.

$$\text{Catalyst activity } K(Hr^{-1}) = -3600/(\text{contact time}) \times \ln((100-\text{propylene conversion rate})/100)$$

wherein in represents logarithm natural.

[Example 1]

A complex metal oxide particle in which 60% by mass of a complex metal oxide whose composition of metal components are represented by $Mo_{12.00}Bi_{0.37}Fe_{1.42}Co_{4.47}Ni_{3.30}Ce_{0.91}Rb_{0.14}$ was carried on a carrier consisting of 40% by mass of silica, that is, a calcined product was used to perform a contacting treatment of propylene, ammonia, and air. It is to be noted that calcined product was prepared by spray-drying a slurry comprising molybdenum, bismuth, iron, cobalt, nickel, cerium, and rubidium to obtain a dried particle, and calcining the dried particle in the air.

The fluidized bed reactor used for the contacting treatment was a vertical cylindrical type reactor having an inner diameter of 8 m and a length of 20 m and comprising an air dispersing plate positioned at 2 m from the bottom and a starting material gas dispersion pipe for supplying propylene and ammonia provided on the air dispersing plate, and the management was performed by determining the reaction temperature from the average value of 12 thermometers, 8 of which are provided on a cross-sectional surface at a height of 5 m from the bottom of the reactor and 4 of which are provided on a cross section at a height of 6 m.

Specifically, the operation was performed as follows.

First, the calcined product was packed into the reactor up to a static bed height of 2.7 m.

After packing, steps A and B as described above were performed.

Next, as condition 1, the flow rate was adjusted so that the molar ratio of ammonia/propylene (N/C) can be 15.00 and the molar ratio of ammonia/air (N/A) can be 0.20.

After the condition 1 was continued for 0.6 hours, as condition 2, the flow rate was set so that the molar ratio of ammonia/propylene (N/C) can be 1.20 and the molar ratio of ammonia/air (N/A) can be 0.10 and held for 3.0 hours, thereby obtaining the catalyst of Example 1.

On that occasion, the reduction rate of the catalyst of Example 1 was 0.65% and the catalyst activity was 7.7.

In addition, acrylonitrile and hydrogen cyanide was produced by ammoxidation reaction by using the catalyst of this Example 1. As a result of analyzing the gas at the reactor outlet, the AN yield was 84.1% and the HCN yield was 3.4%.

[Example 2]

The operation was performed in the same manner as in Example 1 except that the flow rate was set so that the molar ratio of ammonia/propylene in the condition 2 can be 1.00, thereby obtaining the catalyst of Example 2.

On that occasion, the reduction rate of the catalyst of Example 2 was 0.36% and the catalyst activity was 7.6.

In addition, as a result of analyzing the gas at the reactor outlet on that occasion, the AN yield was 84.2% and the HCN yield was 3.3%.

[Example 3]

The operation was performed in the same manner as Example 1 except that the flow rate was set so that the molar ratio of ammonia/propylene in the condition 2 can be 0.85, thereby obtaining the catalyst of Example 3.

On that occasion, the reduction rate of the catalyst of Example 3 was 0.27% and the catalyst activity was 7.4.

In addition, as a result of analyzing the gas at the reactor outlet, on that occasion, the AN yield was 84.2% and the HCN yield was 3.3%.

[Example 4]

The operation was performed in the same manner as Example 1 except that the flow rate was set so that the molar ratio of ammonia/propylene in the condition 2 can be 10.00, thereby obtaining the catalyst of Example 4.

On that occasion, the reduction rate of the catalyst of Example 4 was 0.48% and the catalyst activity was 7.6.

In addition, as a result of analyzing the gas at the reactor outlet, on that occasion, the AN yield was 84.1% and the HCN yield was 3.4%.

[Example 5]

The operation was performed in the same manner as Example 1 except that the flow rate was set so that the molar ratio of ammonia/propylene in the condition 2 can be 3.00, thereby obtaining the catalyst of Example 5.

On that occasion, the reduction rate of the catalyst of Example 5 was 0.22% and the catalyst activity was 7.5.

In addition, as a result of analyzing the gas at the reactor outlet, on that occasion, the AN yield was 84.2% and the HCN yield was 3.3%.

[Example 6]

The operation was performed in the same manner as in Example 1 except that the flow rate was set so that the molar ratio of ammonia/air in the condition 1 can be 0.10, thereby obtaining the catalyst of Example 6.

On that occasion, the reduction rate of the catalyst of Example 6 was 0.21% and the catalyst activity was 7.4.

In addition, as a result of analyzing the gas at the reactor outlet, on that occasion, the AN yield was 84.2% and the HCN yield was 3.2%.

[Example 7]

The operation was performed in the same manner as Example 1 except that the flow rate was set so that the molar ratio of ammonia/air in the condition 1 can be 0.22, thereby obtaining the catalyst of Example 7.

On that occasion, the reduction rate of the catalyst of Example 7 was 0.82% and the catalyst activity was 8.0.

In addition, as a result of analyzing the gas at the reactor outlet, on that occasion, the AN yield was 84.1% and the HCN yield was 3.5%.

[Example 8]

The operation was performed in the same manner as Example 1 except that the flow rate was set so that the molar ratio of ammonia/air in the condition 1 can be 0.24, thereby obtaining the catalyst of Example 8.

On that occasion, the reduction rate of the catalyst of Example 8 was 1.09% and the catalyst activity was 7.9.

In addition, as a result of analyzing the gas at the reactor outlet, on that occasion, the AN yield was 83.8% and the HCN yield was 3.8%.

[Example 9]

The operation was performed in the same manner as Example 1 except that the flow rate was set so that the molar ratio of ammonia/air in the condition 1 can be 0.27, thereby obtaining the catalyst of Example 9.

On that occasion, the reduction rate of the catalyst of Example 9 was 3.60% and the catalyst activity was 8.0.

In addition, as a result of analyzing the gas at the reactor outlet, on that occasion, the AN yield was 83.4% and the HCN yield was 4.1%.

[Example 10]

The operation was performed in the same manner as Example 1 except that the flow rate was set so that the molar ratio of ammonia/air in the condition 1 can be 0.30, thereby obtaining the catalyst of Example 10.

On that occasion, the reduction rate of the catalyst of Example 10 was 4.20% and the catalyst activity was 7.8.

In addition, as a result of analyzing the gas at the reactor outlet, on that occasion, the AN yield was 83.2% and the HCN yield was 4.3%.

[Comparative Example 1]

The operation was performed in the same manner as in Example 1 except that the flow rate was set so that the molar ratio of ammonia/propylene in the condition 1 can be 2.00 and the molar ratio of ammonia/air in the condition 1 can be 0.10, thereby obtaining the catalyst of Comparative Example 1.

On that occasion, the reduction rate of the catalyst of Comparative Example 1 was 0.05% and the catalyst activity was 6.8.

In addition, as a result of analyzing the gas at the reactor outlet, on that occasion, the AN yield was 84.2% and the HCN yield was 2.9%.

[Comparative Example 2]

The operation was performed in the same manner as in Example 1 except that the flow rate was set so that the molar ratio of ammonia/propylene in the condition 1 can be 2.00, thereby obtaining the catalyst of Comparative Example 2.
On that occasion, the reduction rate of the catalyst of Comparative Example 2 was 0.15% and the catalyst activity was 7.0.
In addition, as a result of analyzing the gas at the reactor outlet, on that occasion, the AN yield was 84.2% and the HCN yield was 3.0%.

[Comparative Example 3]

The operation was performed in the same manner as Example 1 except that the flow rate was set so that the molar ratio of ammonia/propylene in the condition 2 can be 8.50, thereby obtaining the catalyst of Comparative Example 3.
On that occasion, the reduction rate of the catalyst of Comparative Example 3 was 5.30% and the catalyst activity was 6.3.
In addition, as a result of analyzing the gas at the reactor outlet, on that occasion, the AN yield was 82.3% and the HCN yield was 4.1%.

[Comparative Example 4]

The operation was performed in the same manner as in Example 1 except that the flow rate was set so that the molar ratio of ammonia/propylene in the condition 2 can be 15.00, thereby obtaining the catalyst of Comparative Example 4.
On that occasion, the reduction rate of the catalyst of Comparative Example 4 was 8.30% and the catalyst activity was 5.1.
In addition, as a result of analyzing the gas at the reactor outlet, on that occasion, the AN yield was 71.0% and the HCN yield was 6.3%.

[Table 1]

| | Ammonia/propylene (N/C) | | Ammonia/air (N/A) | | Reduction rate (%) | AN yield (%) | HCN yield (%) | Catalyst activity |
|---|---|---|---|---|---|---|---|---|
| | Condition 1 | Condition 2 | Condition 1 | Condition 2 | | | | |
| Example 1 | 15.00 | 1.20 | 0.20 | 0.10 | 0.65 | 84.1 | 3.4 | 7.7 |
| Example 2 | 15.00 | 1.00 | 0.20 | 0.10 | 0.36 | 84.2 | 3.3 | 7.6 |
| Example 3 | 15.00 | 0.85 | 0.20 | 0.10 | 0.27 | 84.2 | 3.3 | 7.4 |
| Example 4 | 10.00 | 1.20 | 0.20 | 0.10 | 0.48 | 84.1 | 3.4 | 7.6 |
| Example 5 | 3.00 | 1.20 | 0.20 | 0.10 | 0.22 | 84.2 | 3.3 | 7.5 |
| Example 6 | 15.00 | 1.20 | 0.10 | 0.10 | 0.21 | 84.2 | 3.2 | 7.4 |
| Example 7 | 15.00 | 1.20 | 0.22 | 0.10 | 0.82 | 84.1 | 3.5 | 8.0 |
| Example 8 | 15.00 | 1.20 | 0.24 | 0.10 | 1.09 | 83.8 | 3.8 | 7.9 |
| Example 9 | 15.00 | 1.20 | 0.27 | 0.10 | 3.60 | 83.4 | 4.1 | 8.0 |
| Example 10 | 15.00 | 1.20 | 0.30 | 0.10 | 4.20 | 83.2 | 4.3 | 7.8 |
| Comparative Example 1 | 2.00 | 1.20 | 0.10 | 0.10 | 0.05 | 84.2 | 2.9 | 6.8 |
| Comparative Example 2 | 2.00 | 1.20 | 0.20 | 0.10 | 0.15 | 84.2 | 3.0 | 7.0 |
| Comparative Example 3 | 15.00 | 8.50 | 0.20 | 0.10 | 5.30 | 82.3 | 4.1 | 6.3 |

(continued)

|  | Ammonia/propyl ene (N/C) | | Ammonia/air (N/A) | | Reduction rate (%) | AN yield (%) | HCN yield (%) | Catalyst activity |
|---|---|---|---|---|---|---|---|---|
|  | Conditi on 1 | Conditi on 2 | Conditi on 1 | Conditi on 2 |  |  |  |  |
| Comparative Example 4 | 15.00 | 15.00 | 0.20 | 0.10 | 8.30 | 71.0 | 6.3 | 5.1 |

The present application is based on the Japanese Patent Application (Japanese Patent Application No. 2018-077658) filed on 13 April 2018, the contents of which are incorporated herein by reference.

Industrial Applicability

The catalyst of the present invention has industrial applicability in the production of acrylonitrile and hydrogen cyanide including a step of subjecting propylene to ammoxidation.

## Claims

1. A catalyst comprising molybdenum, bismuth, and iron, wherein
   a reduction rate is in a range of 0.20 to 5.00%.

2. The catalyst according to claim 1, wherein the reduction rate is in a range of 0.70 to 4.30%.

3. The catalyst according to claim 1 or 2, wherein the catalyst comprises a complex metal oxide having a composition represented by the following formula (1):

$$Mo_{12}Bi_aFe_bX_cY_dZ_eO_f \qquad (1)$$

   wherein, X represents at least one element selected from the group consisting of nickel, cobalt, magnesium, calcium, zinc, strontium, barium, and tungsten;
   Y represents at least one element selected from the group consisting of cerium, chromium, lanthanum, neodymium, yttrium, praseodymium, samarium, aluminum, boron, gallium, and indium;
   Z represents at least one element selected from the group consisting of sodium, potassium, rubidium, and cesium;
   a, b, c, d, and e satisfy $0.1{\leq}a{\leq}2.0$, $0.1{\leq}b{\leq}2.8$, $0.1{\leq}c{\leq}0.0$, $0.1{\leq}d{\leq}3.0$, and $0.01{\leq}e{\leq}2.0$, respectively; and
   f represents a number of oxygen atom needed to satisfy an atomic valence requirement of element existing other than oxygen.

4. A method for producing the catalyst according to any one of claims 1 to 3, comprising:

   a contacting step of bringing a calcined product comprising molybdenum, bismuth, and iron into contact with propylene, air, and ammonia, wherein
   the contacting step comprises:

   a step of setting a molar ratio of ammonia/propylene to a condition of greater than 2.50 (condition 1); and
   a step of setting a molar ratio of ammonia/propylene to a condition of 2.50 or less (condition 2).

5. The method for producing the catalyst according to claim 4, wherein
   the molar ratio of ammonia/air in the condition 1 is greater than 0.12, and
   the molar ratio of ammonia/air in the condition 2 is 0.12 or less.

6. A method for producing acrylonitrile comprising a step of reacting propylene, molecular oxygen, and ammonia with each other in the presence of the catalyst according to any one of claims 1 to 3.

7. The method for producing acrylonitrile according to claim 6, wherein the method is carried out by a fluidized bed reactor.

8. The method for producing acrylonitrile according to claim 6 or 7, wherein a molar ratio of ammonia and air to propylene (propylene/ammonia/air) is in a range of 1.0/(0.8 to 2.5)/(7.0 to 12.0).

9. The method for producing acrylonitrile according to any one of claims 6 to 8, wherein a reaction is carried out in a temperature range of 300 to 500°C.

## INTERNATIONAL SEARCH REPORT

| International application No. |
| --- |
| PCT/JP2019/009654 |

### A. CLASSIFICATION OF SUBJECT MATTER

Int. Cl. B01J23/887(2006.01)i, C07C253/26(2006.01)i, C07C255/08(2006.01)i, C07B61/00(2006.01)n

According to International Patent Classification (IPC) or to both national classification and IPC

### B. FIELDS SEARCHED

Minimum documentation searched (classification system followed by classification symbols)
Int. Cl. B01J23/887, C07C253/26, C07C255/08, C07B61/00

Documentation searched other than minimum documentation to the extent that such documents are included in the fields searched
Published examined utility model applications of Japan    1922-1996
Published unexamined utility model applications of Japan  1971-2019
Registered utility model specifications of Japan          1996-2019
Published registered utility model applications of Japan  1994-2019

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 2014-161776 A (ASAHI KASEI CHEMICALS CORP.) 08 September 2014 (Family: none) | 1-9 |
| A | JP 2005-538173 A (ARKEMA) 15 December 2005 & JP 2005-538172 A & US 2006/0128989 A1 & US 2006/0183941 A1 & WO 2004/024666 A1 & WO 2004/024665 A1 & EP 1539669 A1 & EP 1539670 A1 & KR 10-2005-0053643 A & KR 10-2005-0053644 A & CN 1681766 A & CN 1694862 A | 1-9 |
| A | JP 2003-64043 A (ASAHI KASEI CORP.) 05 March 2003 (Family: none) | 1-9 |

☐ Further documents are listed in the continuation of Box C.      ☐ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 17.05.2019 | 28.05.2019 |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
| --- | --- |

Form PCT/ISA/210 (second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017130906 A
- WO 2018211858 A
- JP 2018077658 A